# EUROPEAN PATENT APPLICATION

(11) **EP 2 229 961 A2**
(43) Date of publication of application: **22.09.2010**
(21) Application number: 10460001.0
(22) Date of filing: 15.02.2010
(51) Int. Cl.: A61L 27/12, A61L 27/56

(54) **Method for fabrication of highly porous, calcium phosphate bioactive implant material**

(30) Priority: 17.03.2009 PL 38753009
(71) Applicant: AKADEMIA GORNICZO-HUTNICZA im. Stanislawa Staszica, 30-059 Krakow (PL)
(72) Inventor: Paszkiewicz, Zofia, 30-658 Kraków (PL); Slósarczyk, Anna, 32-091 Michalowice (PL); Zima, Aneta, 30-383 Kraków (PL)
(74) Representative: Magonska, Alina

(57) **Abstract**

The present invention solves the problem of manufacturing bioactive implant material showing particular properties.

In the method according to the invention, the suspension of gelatinous amorphous calcium phosphate synthesized previously by the wet method at Ca/P molar ratio within the range 1.45-1.75 with optional addition of ionic modifiers, preferably CO₃²⁻, Mg²⁺, Mn²⁺, is subjected to aging and sedimentation processes, after which it is decanted and concentrated to the precipitate containing 75-82% of water. The filter cake thus obtained is dried at the temperature of 70-100°C until constant mass, then subjected to mechanical working followed by calcination of the semi-final product obtained at 400-1000°C for 1-5 hours which results in a microporous, mono- or two-phase material of unimodal pore size distribution in the range of 0.05-0.5 µm in the form of granules or shaped articles. In order to obtain granules, the filter cake after drying is crushed into fractions of grain sizes within 0.1-2 mm using appropriate sieves and calcined, whereas in order to obtain shaped implant articles the cake after drying is processed appropriately to get the desired shape and calcined.

## Description

The present invention relates to fabrication of a calcium phosphate bioactive material suitable for filling of bone defects and in manufacturing of drug carriers.

Polish patent No. 154957 discloses a method for the preparation of a ceramic implant material resulting in a pure, reactive, hydroxyapatite powder from which shaped articles intended for implantation can be formed.

Polish patent No. 190486 describes fabrication of highly reactive powders of calcium phosphates involving their one-step precipitation by a slow addition of H₃PO₄ solution to a suspension of Ca(OH)₂, using such amounts of starting compounds to obtain CaO : P₂O₅ molar ratio equal to 1.5-1.66, pH of the reaction medium is maintained within 5-11, temperature within 18-90°C. Simultaneously, the reaction suspension is vigorously stirred. Calcium phosphates thus obtained are in the form of gelatinous, amorphous precipitates in which Ca/P molar ratios are equal to 1.50-1.66. These precipitates are then aged for several dozen hours which causes their transformation to non-stoichiometric hydroxyapatite containing HPO₄²⁻ ions in its structure. After filtering, drying, grinding, the precipitates are calcined at the temperature of 700-900°C which leads to highly reactive powders, being mixtures of HAp and TCP or mono-phase TCP powders.

A method for the fabrication of hydroxyapatite granules is known from Polish patent No. 168078. It comprises preparation of a paste from calcined hydroxyapatite powder of grain size below 60 µm in the amount of 40-70 wt. % and distilled water, its granulation, drying of the granulated product at the temperature increasing up to 100°C at the rate of 5°C per hour, followed by calcination at the temperature of 1250°C for 2 hours.

From US 4629464 patent hydroxyapatite material in granular form, used as a bone substitute is known. It is obtained by mixing hydroxyapatite powder of grain size 0.5 µm with cellulose powder and poly(vinyl alcohol) followed by granulation and calcination at the temperature of ca. 1350°C.

Russian patent No. RU2299869 describes a method of manufacturing ceramic, porous hydroxyapatite-based granules which can be applied in treatment of bone injuries, in orthopedics, in dental surgery. In this method, a suspension of the previously synthesized calcium phosphate powder of Ca/P molar ratio equal to 1.5-1.67 and 10% gelatin solution in the proportion of 0.5-3 ml of the solution and 1 g of the powder is prepared at the temperature of 10-39°C. Then the suspension is dispersed in a natural vegetable oil, stirred using a rotary stirrer. Due to surface tension, spherical granules are formed which are washed, dried and then calcined at the temperature of 900-1250 °C. The product obtained shows controlled grain size and open porosity within 20-80%.

Russian patent RU 2303580 discloses a method for fabricating porous, spherical hydroxyapatite granules of diameter 400-600 µm with bimodal pore size distribution (pores of diameter below 10 µm and above 100 µm). Granules are formed by pressing hydroxyapatite powder and gelatin under the pressure of 10-100 MPa followed by calcination of semi-finished products at the temperature within 900-1250 °C range, while maintaining them at the maximum temperature for 30-300 minutes.

From US2007183955 application a method for preparation of calcium phosphate granules is known. It involves maintaining pH value of the suspension of brushite having a certain particle size distribution in the starting solution below 7 for the sufficient period of time to enable brushite - hydroxyapatite transformation.

European patent EP1380313 describes a method for fabrication of porous particles or granules of α or β tricalcium phosphate, brushite, calcium pyrophosphate, hydroxyapatite or mixtures thereof. The method involves mixing of self-setting calcium phosphate cement powder and gelatin powder in the proportion of 3 : 0.25 to 1 and adding Na₂HPO₄ solution. The paste thus formed is immediately placed in a syringe and after several minutes morsels are squeezed out of the syringe. The morsels are soaked in distilled water at the temperature of 37°C for several days in order to dissolve gelatin and allow formation of mutually interconnected pores. After that, thermal treatment aimed at burning out organic components followed by successive cooling to room temperature is performed. Then calcined, sintered morsels are crushed and sieved in order to obtain porous granules.

Using the known methods, however, microporous materials of controlled pore size distribution and pore sizes within the range of hundredth or tenth parts of micrometer can not be prepared. Hence, these materials are of limited applications.

Preparation of calcium phosphate implant materials according to the present invention comprises aging and sedimentation of a slurry of gelatinous, amorphous calcium phosphate synthesized previously by a wet method and showing the Ca/P molar ratio in the range of 1.45-1.75 with optional addition of ionic modifiers, preferably CO₃²⁻, Mg²⁺, Mn²⁺, followed by decantation and concentration to the precipitate containing 75-82% of water. A filter cake thus obtained is dried until constant mass at the temperature of 70-100°C followed by mechanical working and calcination of the semi-final product obtained at 400-1000°C during the period of 1-5 hours which results in a biocompatible, microporous mono- or two-phase material exhibiting open porosity within the range of 50-70% and uni-modal pore size distribution in the range of 0.05-0.5 µm in the form of granules or shaped articles. In order to fabricate granules, a filter cake after drying is crushed and sieved into grain fractions of sizes within 0.1-2 mm and then calcined, whereas in order to produce shaped implant articles the cake is processed in the appropriate way in order to get the desired shape and calcined.

The method according to the invention allows to obtain microporous hydroxyapatite, modified hydroxyapatite, hydroxyapatite-whitlockite or whitlockite material characterized by open porosity in the range of 50-70% and uni-modal pore size distribution within 0.05-0.5 µm. Such pore size distribution makes it possible to apply this material as an independent implant material in bone substitution, as a drug carrier, as well as a component of composite implant materials.

Material fabricated according to the invention shows high water absorption ability, which is a particularly advantageous feature in medical applications.

### Example 1

Suspension of calcium phosphate prepared previously by a known method (e.g. using the procedure described in Polish patent No. 154957) of Ca/P molar ratio equal to 1.67 is subjected to aging and sedimentation processes during the period of 2 days at room temperature, after which it is decanted and concentrated by centrifugation to get the precipitate showing 80% water content. The filter cake obtained is dried till constant mass at the temperature of 90°C, crushed in a mortar in order to get the grain sizes below 0.6 mm. A fraction of sizes between 0.3-0.5 mm isolated using the relevant sieves is subjected to thermal treatment at 800°C for 4 hours. Hydroxyapatite (HAP) granules thus obtained of sizes between 0.3-0.5 mm are washed with distilled water in order to remove dusty fractions, then dried at 100°C until constant mass. Granules exhibit open porosity P_{c} of 68%, average pore size of 0.1 µm and high ability to absorb humidity (body fluids, drug in the form of solutions).

### Example 2.

Suspension of amorphous calcium phosphate prepared according to the method disclosed in Polish patent No. 190486 at Ca/P molar ratio in the starting reagents equal to 1.6 is subjected to aging and sedimentation processes followed by concentration in a centrifuge to get a dense precipitate containing 78% of water and drying at the temperature of 90°C until constant mass. From the dried body, articles of desired shapes intended for implantation are cut. Then they are calcined at 900°C for 2 hours. Shaped two-phase hydroxyapatite-whitlockite (HAP-TCP) implant articles thus fabricated are washed with ethyl alcohol in order to remove dusty fractions and then dried at the temperature of 100°C till constant mass.

The material exhibits open porosity P_{c} of 63% and average pore size of 0.2 µm.

### Example 3.

Suspension of amorphous calcium phosphate prepared by the wet method in the presence of CO₃²⁻ ions at Ca/P molar ratio in the starting reagents equal to 1.70 is subjected to aging, sedimentation and decantation processes followed by concentration in a centrifuge to a precipitate containing 82% of water. The body thus obtained is dried at 80°C until constant mass and crushed in a mortar to get the grains of sizes below 1.2 mm. A fraction of sizes within 0.4-1.0 mm is separated using appropriate sieves and it is calcined at 600°C for 1 hour. Irregular granules based on carbonated hydroxyapatite (CHAp) of sizes between 0.4-1.0 mm are washed with distilled water and then dried at 100°C until constant mass.

The material shows open porosity P_{c} of 63% and average pore size of 0.15 µm.

## Claims

1. A method for fabrication of highly porous calcium phosphate bioactive implant material based on the wet synthesis of calcium phosphates **characterized in that** the suspension of gelatinous amorphous calcium phosphate synthesized previously by the wet method at Ca/P molar ratio within the range 1.45-1.75 with optional addition of ionic modifiers, preferably CO₃²⁻, Mag²⁺, Mn²⁺, is subjected to aging and sedimentation processes, after which it is decanted and concentrated to the precipitate containing 75-82% of water, then the filter cake thus obtained is dried at the temperature of 70-100°C, then subjected to mechanical treatment followed by calcination of the semi-final product obtained at 400-1000°C for 1-5 hours which results in a microporous, mono- or two-phase material of unimodal pore size distribution in the range of 0.05-0.5 µm.

2. A method according to claim 1 **characterized in that** the filter cake after drying is crushed and the granules formed are fractionated using appropriate sieves into fractions of grain sizes within the range of 0.1-2 mm.

3. A method according to claim 1 **characterized in that** the filter cake after drying is processed in the appropriate way to get the desired shape in order to obtain a shaped implant article and then calcined.
